# EUROPEAN PATENT SPECIFICATION

(11) **EP 2 003 967 B1**
(45) Date of publication and mention of the grant of the patent: **07.09.2011**
(21) Application number: 07753923.7
(22) Date of filing: 23.03.2007
(51) Int. Cl.: A01N 37/18, G01N 33/574, C07K 5/10, G01N 33/78

(54) **USE OF SOMATOSTATIN AGONISTS TO TREAT MEDULLARY THYROID CARCINOMA**
VERWENDUNG VON SOMATOSTATIN-AGONISTEN ZUR BEHANDLUNG VON MEDULLÄREM SCHILDDRÜSENKARZINOM
UTILISATION D'AGONISTES DE SOMATOSTATINE POUR TRAITER UN CARCINOME MÉDULLAIRE THYROÏDIEN

(30) Priority: 23.03.2006 US 785399 P; 23.03.2006 US 785077 P; 28.03.2006 US 786814 P
(43) Date of publication of application: 24.12.2008
(73) Proprietor: IPSEN PHARMA, 92100 Boulogne-Billancourt (FR); Universita' degli Studi di Ferrara, 44100 Ferrara (IT)
(72) Inventor: ZATELLI, Maria, Chiara, I-44100 Ferrara (IT); CULLER, Michael, Dewitt, Hopkinton, MA 01748 (US); DEGLI UBERTI, Ettore, C., I-44100 Ferrara (IT)
(74) Representative: Harrison Goddard Foote
(86) International application number: PCT/US2007/007339
(87) International publication number: WO 2007/112045

(56) References cited:
- WO-A2-02/070555
- ZATELLI M C ET AL: "Somatostatin receptors: From basic science to clinical approach - Thyroid" DIGESTIVE AND LIVER DISEASE 200402 US, vol. 36, no. SUPPL. 1, February 2004 (2004-02), pages S86-S92, XP002535480 ISSN: 1590-8658
- VAINAS I G ET AL: "The therapeutic value of SST-A octreotide alone or with adjuvant treatment in patients with advanced medullary thyroid carcinoma and positive <111>In-octreotide scan" HELLENIC JOURNAL OF NUCLEAR MEDICINE,, vol. 8, no. 1, 1 January 2005 (2005-01-01), pages 43-47, XP008107757
- ZINK A ET AL: "Inhibitory effect of somatostatin on cAMP accumulation and calcitonin secretion in C-cells: Involvement of pertussis toxin-sensitive G-proteins" MOLECULAR AND CELLULAR ENDOCRINOLOGY, ELSEVIER IRELAND LTD, IE, vol. 86, no. 3, 1 August 1992 (1992-08-01), pages 213-219, XP023488908 ISSN: 0303-7207 [retrieved on 1992-08-01]
- ZATELLI M C ET AL: "Somatosratin, but not somatostatin receptor subtypes 2 and 5 selective agonists, inhibits calcitonin secretion and gene expression in the human medullary thyroid carcinoma cell line, TT" HORMONE AND METABOLIC RESEARCH, THIEME-STRATTON, STUTTGART, DE, vol. 34, no. 5, 1 May 2002 (2002-05-01), pages 229-233, XP008107756 ISSN: 0018-5043
- VAINAS I ET AL: "Somatostatin receptor expression in vivo and response to somatostatin analog therapy with or without other antineoplastic treatments in advanced medullary thyroid carcinoma" JOURNAL OF EXPERIMENTAL AND CLINICAL CANCER RESEARCH, ROME, IT, vol. 23, no. 4, 1 December 2004 (2004-12-01), pages 549-559, XP008107762 ISSN: 0392-9078
- ZATELLI M C ET AL: "Somatostatin receptor subtype 1-selective activation reduces cell growth and calcitonin secretion in a human medullary thyroid carcinoma cell line" BIOCHEMICAL AND BIOPHYSICAL RESEARCH COMMUNICATIONS 2002 US, vol. 297, no. 4, 2002, pages 828-834, XP002535481 ISSN: 0006-291X
- VITALE ET AL.: 'Slow Release Lanreotide in Combination with Interferon-a2b in the Treatment of Symptomatic Advanced Medullary Thyroid Carcinoma' J. CLIN. ENDOCRIN. MET. vol. 85, 2000, pages 983 - 988, XP008130972
- ZATELLI ET AL.: 'Somatostatin Receptor Subtype 1-Selective Activatin Reduces Cell Growth and Calcitonin Secretion in a Human Medullary Thyroid Carcinoma Cell Line' BIOCHEM. BIOPHYS. RES. COMM. vol. 297, 2002, pages 828 - 834, XP002535481
- DANILA ET AL.: 'Somatostain Receptor-Specific Analogs: Effects on Cell Proliferation and Growth Hormone Secretion in Human Somatotroph Tumors' J. CLIN. ENDOCRIN. MET. vol. 86, 2001, pages 2976 - 2981, XP008130973

## Description

### BACKGROUND OF THE INVENTION

Somatostatin, a tetradecapeptide discovered by Brazeau et al. (Science, 1973, 179:77-79), has been shown to have potent inhibitory effects on various secretory processes and cell proliferation in normal and neoplastic human tissues such as pituitary, pancreas and the gastrointestinal tract. Somatostatin also acts as a neuromodulator in the central nervous system. These biological effects of somatostatin, all inhibitory in nature, are elicited through a series of G protein coupled receptors, of which five different subtypes have been characterized, hereinafter referred to as "SSTR-1", "SSTR-2", "SSTR-3", "SSTR-4" and "SSTR-5" for each of the five receptors or generally and/or collectively as "SSTR" (Patel, Y. C., Front. Neuroendocrinol., 1999, 20:157-98; and Zatelli, M. C. et al., J. Endocrinol. Invest., 2004, 27 Suppl(6):168-70). These five subtypes have similar affinities for the endogenous somatostatin ligands but have differing distribution in various tissues. Somatostatin binds to the five distinct receptor subtypes with relatively high and equal affinity for each.

There is evidence that somatostatin regulates cell proliferation by arresting cell growth via SSTR-1, -2, -4 and -5 subtypes (Buscail, L. et al., Proc. Natl. Acad. Sci. USA, 1995, 92:1580- 4; Buscail, L. et al., Proc. Natl. Acad. Sci. USA, 1994, 91:2315-9; Florio, T. et al., Mol. Endocrinol., 1999, 13:24-37; and Sharma, K. et al., Mol. Endocrinol., 1999, 13:82-90) or by inducing apoptosis via SSTR-3 subtype (Sharma, K. et al., Mol. Endocrinol., 1996, 10:1688-96). Somatostatin and various analogues have been shown to inhibit normal and neoplastic cell proliferation *in vitro* and *in vivo* (Lamberts, S. W. et al., Endocrin. Rev., 1991, 12:450-82) via specific somatostatin receptors. (Patel, Y. C., Front Neuroendocrin., 1999, 20:157-98) and possibly different postreceptor actions (Weckbecker, G. et al., Pharmacol. Ther., 1993, 60:245-64; Bell, G. I. and Reisine, T., Trends Neurosci., 1993, 16:34-8; Patel, Y. C. et al., Biochem. Biophys. Res. Comm., 1994, 198:605-12; and Law, S. F. et al., Cell Signal, 1995, 7:1-8). In addition, there is evidence that distinct SSTR subtypes are expressed in normal and neoplastic human tissues (Virgolini, I. et al., Eur. J Clin. Invest., 1997, 27:645-7) conferring different tissue affinities for various somatostatin analogues and variable clinical response to their therapeutic effects.

Binding to the different types of SSTR subtypes has been associated with the treatment of various conditions and/or diseases. For example, the inhibition of growth hormone has been attributed to SSTR-2 (Raynor, et al., Molecular Pharmacol., 1993, 43:838; and Lloyd, et al., Am. J. Physiol., 1995, 268:G102) while the inhibition of insulin has been attributed to SSTR-5. Activation of SSTR-2 and SSTR-5 has been associated with growth hormone suppression and more particularly GH secreting adenomas (acromegaly) and TSH secreting adenomas. Activation of SSTR-2 but not SSTR-5 has been associated with treating prolactin secreting adenomas. Other indications associated with activation of the somatostatin receptor subtypes include inhibition of insulin and/or glucagon for treating diabetes mellitus, angiopathy, proliferative retinopathy, dawn phenomenon and nephropathy, inhibition of gastric acid secretion and more particularly peptic ulcers, enterocutaneous and pancreaticocutaneous fistula, irritable bowel syndrome, Dumping syndrome, watery diarrhea syndrome, AIDS related diarrhea, chemotherapy-induced diarrhea, acute or chronic pancreatitis and gastrointestinal hormone secreting tumors, treatment of cancer such as hepatoma, inhibition of angiogenesis, treatment of inflammatory disorders such as arthritis, retinopathy, chronic allograft rejection, angioplasty, preventing graft vessel and gastrointestinal bleeding. It is preferred to have an analog which is selective for the specific Somatostatin receptor subtype or subtypes responsible for the desired biological response, thus reducing interaction with other receptor subtypes which could lead to undesirable side effects.

Somatostatin and its receptors are expressed in normal human parafollicular C cells. Medullary thyroid carcinoma (MTC) is a tumor originating from thyroid parafollicular C cells that produces somatostatin as well as calcitonin and several other peptides (Moreau, J.-P. et al., Metabolism, 1996, 45(8 Suppl.1):24-6). This hypersecretion can result in a syndrome very similar to carcinoid syndrome in which the patients may experience diarrhea, flushing, swelling, asthenia, weight loss, malaise and pain. MTC can occur sporadically or as part of the multiple endocrine neoplasia type-2 syndromes and familial MTC (Santoro, M. et al., J. Intern. Med., 1998, 243:505-8) representing 5-10% of all thyroid cancers (Cascon, A. et al., J. Clin. Endocrinol. Metab., 2005, 90:2127-30).

To date, the most effective treatment for MTC is total thyroidectomy associated with central neck lymph node dissection (Vitale, G. et al., Cancer, 2001, 91:1797-1808). While the complication rate for the initial surgery is reported to be relatively low at approximately 2%, second or additional neck dissections are associated with a very high rate of permanent damage to the patient in the form of hypoparathyroidism and laryngeal nerve palsy. In addition, persistent or recurrent disease is reported in 29-85% of the cases (Beressi, N. et al., Thyroid, 1998, 8:1039-44; Gimm, O. et al., World J. Surg., 1998, 22:562-8). The quality of life issues resulting from surgical complications are so severe that initial neck dissections are probably often performed too conservatively, accounting for the relatively high reported safety rate, and clinicians are often reluctant to perform additional surgeries. Radiotherapy and chemotherapy are of limited value in advanced MTC.

In 2006, the estimated prevalence in the United States and Europe for unresolved, symptomatic MTC was approximately 5,200 patients with an annual incidence rate in both territories of approximately 440 patients per year. There are currently no effective medical therapies for suppression of either calcitonin secretion or MTC proliferation following failed surgery, even though the symptoms, as well as the causative agents, are clear and readily measurable. Since there are no other approved treatments, any improvement would be beneficial, and as such, there is a major unmet medical need for a medical treatment for symptomatic residual medullary thyroid carcinoma following initial surgery. There is evidence of a highly variable SSTR expression pattern in MTC (Reubi, J. C. et al., Lab. Invest., 1991, 64:567-73; Zatelli, M. C. et al., Dig. Liver Dis., 2004, 36 Suppl. 1:S86-92). Previous *in vitro* studies of the effect of receptor subtype selective somatostatin analogs on the proliferation and calcitonin production/secretion by the MTC TT cell line, which expresses all SSTR subtypes, indicated that somatostatin and selective SSTR agonists do not all affect cell proliferation and calcitonin secretion the same (Zatelli, M. C. et al., J. Clin. Endocrinol. Metab., 2001, 86:2161-9; Zatelli, M. C. et al., Biochem. Biophys. Res. Common., 2002, 297:828-34).

The aforementioned *in vitro* studies of the TT MTC cell line indicated that SSTR-2 ligands suppressed the proliferation of TT cells, while SSTR-5 ligands antagonized and reversed the effects of activity at SSTR-2. It has been reported that activity at neither SSTR-2 nor SSTR-5, either individually or in combination, is effective in suppressing either calcitonin expression or secretion. These results may explain the lack of efficacy in suppressing calcitonin secretion of the two commercially available somatostatin analogs, lanreotide and octreotride, both of which have been shown to be highly potent SSTR-2 selective agonists with moderate SSTR-5 activity. In addition, treatment of MTC with either lanreotide or octreotide have had little effect on reducing tumour size and/or increasing patient survival rate (Diez, J.J. et al., J. Endocrinol. Invest., 2002, 25:773-8; see also Vitale, G. et al., J. Clin. Endocrinol. Metab., 2000, 85:983-8).

Vainas et al. (Hell. J Nucl med 2005; 8(1); 43-47) discuss the therapeutic value of the somatostatin analogue octreotide in patients with medullary carcinoma.

Zink et al. (Molecular and Cellular Endocrinology, 86, (1992), 213-219) discuss the role of pertussis toxin-sensitive G-proteins in the inhibitory effect of somatostatin on cAMP accumulation and calcitonin secretion in the C-cells of a rat medullary thyroid carcinoma cell line.

Zatelli et al. (Horm. Metab. Res.; 2002; 34; 229-233) describes the effect of somatostatin, SSTR-2 or SSTR-5 agonists upon CT secretion and gene expression in TT cells. Zatelli *et al.* concluded that while somatostatin decreased CT secretion and CT gene expression, the SSTR-2 and SSTR-5 agonists tested did not modify CT secretion or gene expression.

Vainas et al. (J. Exp. Clin. Cancer Res.; 23; 4, 2004; 549-559) describe the evaluation of somatostatin analogues in patients with persistent or relapsed medullary thyroid carcinoma. Daily administration of octreotide and monthly administration of long acting octreotide provided a subjective and biological partial remission in a proportion of patients.

WO 02/070555 is directed to methods of reducing the rate of proliferation of medullary thyroid carcinoma cells by the application of SSTR-2 agonists. WO 02/070555 is further directed to a method of modulating the rate of proliferation of medullary thyroid carcinoma cells by the application of a combination of SSTR-2 and SSTR-5 agonists, where the SSTR-5 agonist is effective in attenuating the effect of the SSTR-2 agonist.

Danila et al. (J. Clin. Endocrin. Met.; 86 (2001); 2976-2981) researched the effects of somatostatin, lanreotide and an SSTR-2 or SSTR-5 agonist upon the cellular proliferation of and growth hormone secretion of somatotroph pituitary adenomas in primary cultures. Danila *et al.* disclose that somatostatin and analogs thereof could inhibit cellular proliferation and, in a non-correlative manner, decrease the secretion of growth hormones.

Vitale et al. (Clin. Endocrin. Met.; 85; 2000; 983-988) reports upon the effects of a combination of a somatostatin analog and interferon-a2b to ameliorate the symptoms of medullary thyroid carcinoma in seven patients with advanced cancers. The drug combination delivered relief from clinical symptoms to 6 of the 7 patients and was well tolerated.

Continuing studies have revealed that SSTR-1 analogs exhibit antiproliferative properties while also effectively suppressing calcitonin expression and secretion of MTC. It has also been observed that SSTR-1 selective analogs are also effective in suppressing the phosphorylation of cAMP response element binding protein, a transcription factor that facilitates a number of secretory pathways. These observations regarding the SSTR-1 receptor suggest that it too may effectively curtail hypersecretory action of MTC cells. It has also been observed that SSTR-1 selective agonists are more effective in suppressing the hypersecretions MTC than native somatostatin which activates all of the receptor subtypes. As observed with the antagonistic interaction between the SSTR-2 and SSTR-5 receptors, certain receptor subtypes activated by native somatostatin may antagonise other subtypes.

It is apparent that distinct somatostatin receptor subtypes influence cell growth rate and hormone secretion differently, suggesting that the human body's response to a particular somatostatin analog is highly tissue specific. Development and assessment of SSTR subtype analogues selective on MTC cell growth would provide a useful tool for clinical application. The previously discussed *in vitro* studies conducted using MTC cell line TT provided valuable insight as to the ability of different somatostatin and somatostatin analogs to suppress the proliferation of and to reduce the secretion of calcitonin by MTC TT cells *in vitro.* To further extend the study and search for treatments for MTC, it would be beneficial to obtain and evaluate primary cultures of human thyroid medullary carcinoma cells, as well as other thyroid follicular cell tumours, removed at first surgery from actual patients.

### SUMMARY OF THE INVENTON

As discussed herein, for the first time, the responsiveness of primary MTC tissue taken directly from human patients was studied in an effort to provide results useful to develop efficient and effective clinical treatments for human patients suffering from MTC. In particular, Applicants demonstrate: 1) that TT cell lines behave in a manner which is different than that of primary MTC cells isolated from tumor samples; and 2) that primary tumor samples from different patients also differ in their response to various somatostatin agonists. Taken together, this information provides a basis for determining optimum treatment plans for patients suffering from MTC, particularly post-operative reoccurrence of the disease, and its symptoms.

Applicants discovered that primary MTC tumor cells can be subdivided into two classifications according to inhibition of calcitonin secretion by the clinically available somatostatin analog "lanreotide: "lanreotide responsive", in which calcitonin secretion was reduced by about 15% or more, or "lanreotide non-responsive"", in which calcitonin secretion was reduced by about 15% or less.

In the "lanreotide responsive" group, calcitonin secretion was significantly (P<0.05) reduced by lanreotide (-27%), the SSTR-1 selective agonist Caeg-c[D-Cys-3-Pal-D-Trp-Lys-D-Cys]-Thr(Bzl)-Tyr-NH₂ (-17%), the SSTR-2 selective agonist c[Tic-Tyr-D-Trp-Lys-Abu-Phe] (-23%), and the SSTR-5 selective agonist D-Phe-Phe-Trp-D-Trp-Lys-Thr-Phe-Thr-NH₂ (-28%). A direct correlation was observed between the inhibitory effect of SSTR-2 interacting analogs and SSTR-2 mRNA expression levels. The SSTR-1 and SSTR-2 selective agonists had an additive inhibitory effect on calcitonin secretion (-38%) in this group. Little to no effect on cell viability was observed after treatment with any SSTR agonist. Treatment with a combination of an SSTR-1 agonist and an SSTR-2 agonist would be highly efficacious in suppressing calcitonin in the "lanreotide-responsive" subclass.

In the "lanreotide non-responsive" group, however, neither SSTR-2 nor SSTR-5 selective agonists alter calcitonin secretion. Only SSTR-1 selective agonists significantly reduce calcitonin secretion (-13%;P<0.05). In the "lanreotide non-responsive" group, cell viability was significantly (P<0.05) reduced by both lanreotide (-14%) and by the SSTR-2 selective agonist, c[Tic-Tyr-D-Trp-Lys-Abu-Phe] (-13%), but not by the SSTR-1 selective agonist, Caeg-c[D-Cys-3-Pal-D-Trp-Lys-D-Cys]-Thr(Bzl)-Tyr-NH2 or by the SSTR-5 selective agonist, D-Phe-Phe-Trp-D-Trp-Lys-Thr-Phe-Thr-NH₂.

CgA secretion was not affected by somatostatin analogs in any of the cultures.

In a first aspect, the invention is directed to a method of determining the treatment for a patient suffering from medullary thyroid carcinoma comprising the steps of:
a) administering a somatostatin agonist or a pharmaceutically acceptable salt thereof to a sample of said medullary thyroid carcinoma obtained from said patient;
b) measuring the change in calcitonin secreted by said sample after said administration; and
c) determining a treatment,
wherein said determining of said treatment is based upon said identified change in calcitonin secretion.

The somatostatin agonist useful to practice the method of the first aspect of the invention includes SSTR-2 agonists or SSTR-2 selective agonists or pharmaceutically acceptable salts thereof. Exemplary SSTR-2 agonists include, but are not limited to D-β-Nal-c[Cys-Tyr-D-Trp-Lys-Val-Cys]-Thr-NH₂, D-Phe-Cys-Phe-D-Trp-Lys-Thr-Cys-Thr-ol, Dop2-D-Lys(Dop2)-c[Cys-Tyr-D-Trp-Lys-Abu-Cys]-Thr-NH₂, c[Tic-Tyr-D-Trp-Lys-Abu-Phe], 4-(2-Hydroxyethyl)-1-piperazinylacetyl-D-Phe-c[Cys-Tyr-D-Trp-Lys-Abu-Cys]-Thr-NH₂ or 4-(2-Hydroxyethyl)-l-piperazine-2-ethanesulfonyl-D-Phe-c[Cys-Tyr-D-Trp-Lys-Abu-Cys]-Thr-NH₂, or pharmaceutically acceptable salts thereof. In one feature of the method of determining the treatment for a patient suffering from medullary thyroid carcinoma, the selection of treatment is based upon the change in calcitonin levels following treatment with an SSTR-2 agonist where the choice of treatment for carcinomas or patients exhibiting a decrease in calcitonin secretion of less than about 15% (i.e. a "non-responder" carcinoma or patient) may differ from the choice of treatment for carcinomas or patients exhibiting a decrease in calcitonin secretion of greater than about 15% (i.e. a "responder" carcinoma or patient). In another aspect, the MTC patient in need of treatment and benefiting from the method of the instant invention has undergone at least one thyroidectomy.

### BRIEF DESCRIPTION OF THE DRAWINGS

- **Figure 1:**: SSTR Expression in Medullary Thyroid Carcinoma
Qualitative RT-PCR was performed on 36 MTC samples, showing the presence or absence of the expression of each SSTR. The bars indicate the percentage of positive samples.
- **Figure 2**: Effect of SSTR Agonists on Calcitonin Secretion by Medullary Thyroid Carcinoma

Primary cultured cells from 18 selected MTC samples were incubated in ninety six well plates for six hours with each SSTR agonist at 10⁻⁸ M. Control cells were treated with vehicle solution. Calcitonin levels in the conditioned medium were measured by immunoradiometric assay (IRMA) among eight replicates in the conditioned medium from treated and untreated (control) primary cultured cells. As described herein, samples were divided according to calcitonin secretion inhibition after treatment with lanreotide in group A (responders, black bars) and group B (non-responders, white bars). Data are expressed as the mean ± SE percent hormone secretion inhibition vs. untreated control cells. *P<0.05 and **P<0.01 vs. control.

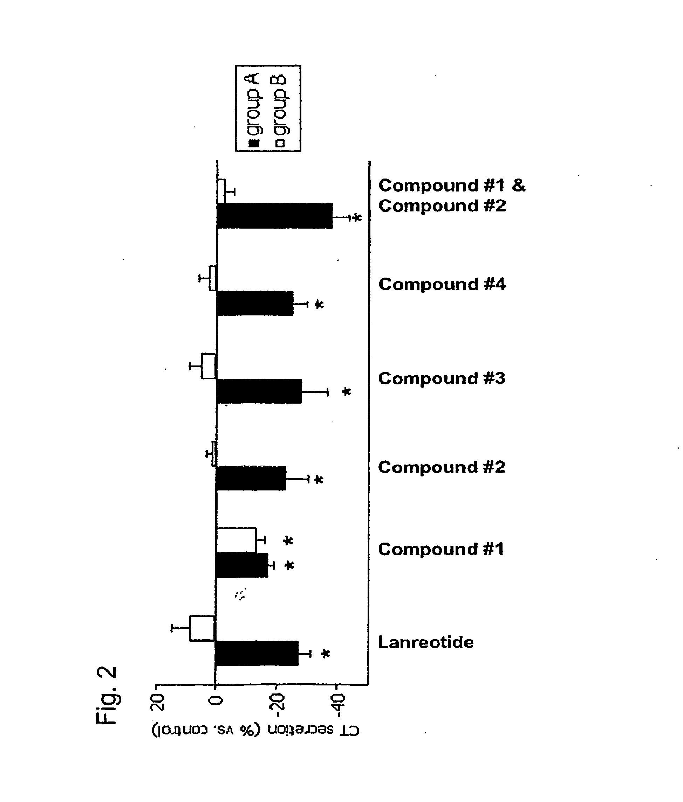

The compounds represented in Figure 2 are as follows:
Compound #1: Caeg-c[D-Cys-3-Pal-D-Trp-Lys-D-Cys]-Thr(Bzl)-Tyr-NH₂
Compound #2: c[Tic-Tyr-D-Trp-Lys-Abu-Phe]
Compound #3: D-Phe-Phe-Trp-D-Trp-Lys-Tbr-Phe-Thr-NH₂
Compound #4: D-Phe-[Cys-Tyr(I)-D-Trp-Lys-Val-Cys]-Thr-NH₂

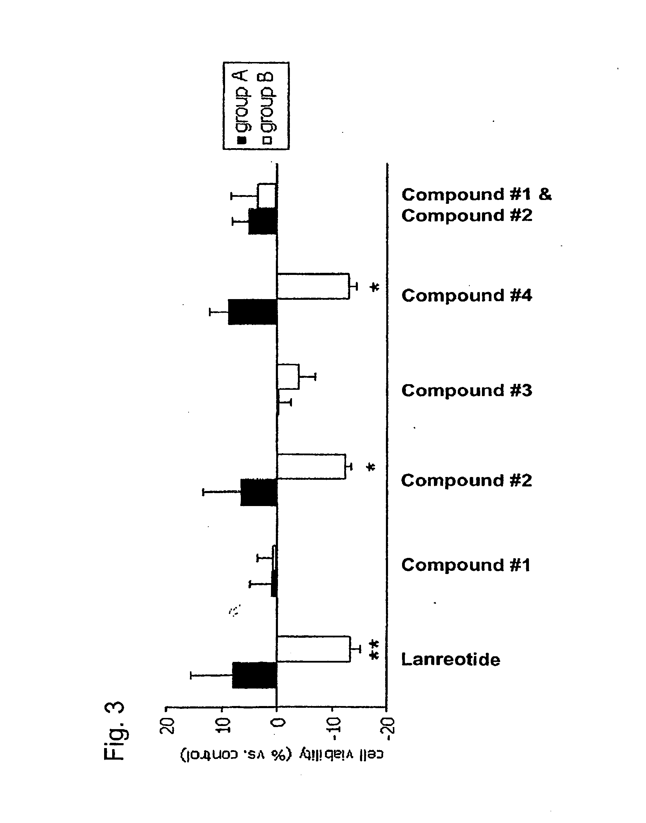

- **Figure 3:**: Effect of SSTR Agonists on Cell Viability of Medullary Thyroid Carcinoma Primary Cultures

Primary cultured cells from 18 selected MTC samples were incubated in ninety six well plates for forty eight hours with each SSTR agonist at 10⁻⁸ M. Control cells were treated with vehicle solution. Cell viability was measured by a colorimetric method among eight replicates from treated and untreated (control) primary cultured cells. As described herein, samples were divided according to calcitonin secretion inhibition after treatment with lanreotide in group A (responders, black bars) and group B (non responders, white bars). Data are expressed as the mean ± SE percent cell viability reduction vs. untreated control cells. *P<0.05, and **P<0.01 vs. control.

The compounds represented in Figure 3 are as follows:
Compound #1: Caeg-c[D-Cys-3-Pal-D-Trp-Lys-D-Cys]-Thr(Bzl)-Tyr-NH2
Compound #2: c[Tic-Tyr-D-Trp-Lys-Abu-Phe]
Compound #3: D-Phe-Phe-Trp-D-Trp-Lys-Thr-Phe-Thr-NH₂
Compound #4: D-Phe-[Cys-Tyr(I)-D-Trp-Lys-Val-Cys]-Thr-NH₂.

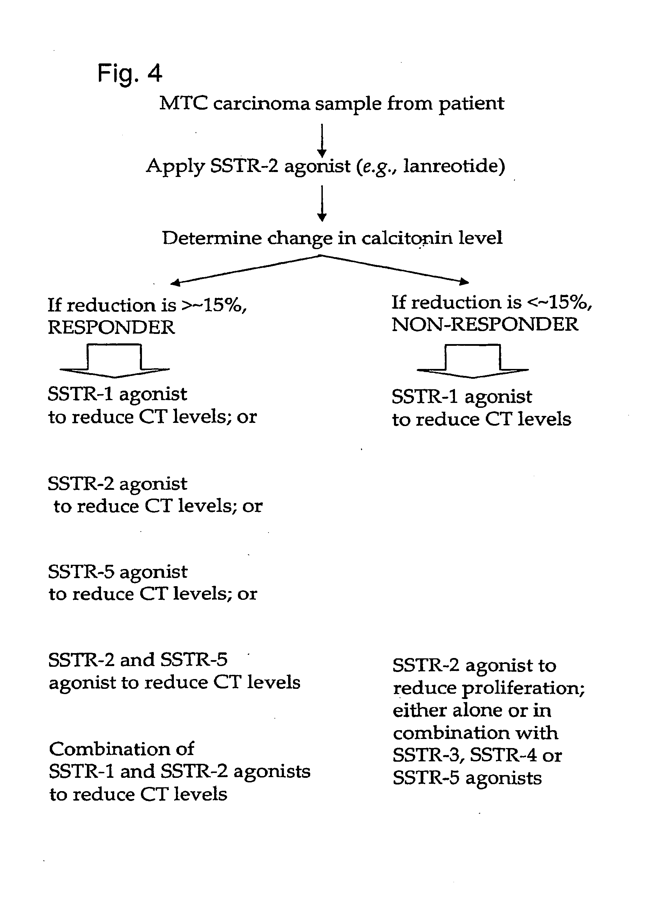

- **Figure 4:**: Schematic of an embodiment of the invention.

### DETAILED DESCRIPTION OF THE INVENTION

It is believed that one skilled in the art can, based on the description herein, utilize the present invention to its fullest extent. The following specific embodiments are, therefore, to be construed as merely illustrative, and not limitative of the remainder of the disclosure in any way whatsoever.

Unless defined otherwise, all technical and scientific terms used herein have the same meaning as commonly understood by one of ordinary skill in the art to which this invention belongs.

Various somatostatin receptors have been isolated, *e.g*., SSTR-1, SSTR-2, SSTR-3, SSTR-4, and SSTR-5. Thus, a somatostatin agonist may be one or more of an SSTR-1 agonist, SSTR-2 agonist, SSTR-3 agonist, SSTR-4 agonist or a SSTR-5 agonist.

What is meant by an SSTR-1 receptor agonist (i.e., SSTR-1 agonist) is a compound which has a high binding affinity (*e.g*., Ki of less than 100 nM or preferably less than 10 nM or less than 1 nM) for SSTR-1 (*e.g*., as defined by the receptor binding assay in U.S. Patent No. 7,084,117 incorporated herein by reference in its entirety). What is meant by an SSTR-1 receptor selective agonist is an SSTR-1 receptor agonist which has a higher binding affinity (*i.e.,* lower Ki) for SSTR-1 than for another receptor, i.e., SSTR-2, SSTR-3, SSTR-4 or SSTR-5.

What is meant by an SSTR-2 receptor agonist is a somatostatin agonist which has a high binding affinity (*e.g*., Ki of less than 100 nM or preferably less than 10 nM or less than 1 nM) for SSTR-2 (*e.g*., as defined by the receptor binding assay in U.S. Patent No. 7,084,117 incorporated herein by reference in its entirety). What is meant by an SSTR-2 receptor selective agonist is an SSTR-2 receptor agonist which has a higher binding affinity (*i.e*., lower Ki) for SSTR-2 than for any other somatostatin receptor i.e., SSTR-1, SSTR-3, SSTR-4 or SSTR-5.

What is meant by an SSTR-3 receptor agonist is a somatostatin agonist which has a high binding affinity (*e.g*., Ki of less than 100 nM or preferably less than 10 nM or less than 1 nM) for SSTR-3 (*e.g*., as defined by the receptor binding assay in U.S. Patent No. 7,084,117 incorporated herein by reference in its entirety). What is meant by an SSTR-3 receptor selective agonist is an SSTR-3 receptor agonist which has a higher binding affinity (*i.e*., lower Ki) for SSTR-3 than for any other somatostatin receptor i.e., SSTR-1, SSTR-2, SSTR-4 or SSTR-5.

What is meant by an SSTR-4 receptor agonist is a somatostatin agonist which has a high binding affinity (*e.g*., Ki of less than 100 nM or preferably less than 10 nM or less than 1 nM) for SSTR-4 (*e.g*., as defined by the receptor binding assay in U.S. Patent No. 7,084,117). What is meant by an SSTR-4 receptor selective agonist is an SSTR-4 receptor agonist which has a higher binding affinity (*i.e*., lower Ki) for SSTR-4 than for any other somatostatin receptor i.e., SSTR-1, SSTR-2, SSTR-3 or SSTR-5.

What is meant by an SSTR-5 receptor agonist is a somatostatin agonist which has a high binding affinity (*e.g*., Ki of less than 100 nM or preferably less than 10 nM or less than 1 nM) for SSTR-5 (*e.g*., as defined by the receptor binding assay in U.S. Patent No. 7,084,117). What is meant by an SSTR-5 receptor selective agonist is an SSTR-5 receptor agonist which has a higher binding affinity (*i.e*., lower Ki) for SSTR-5 than for any other somatostatin receptor i.e., SSTR-1, SSTR-2, SSTR-3 or SSTR-4.

Some somatostatin agonist compounds exhibit high binding affinities for two, or even three, somatostatin receptors as compared to other somatostatin receptors. Such somatostatin agonists are also classified as a somatostatin agonist wherein the compound has a high binding affinity (*e.g*., Ki of less than 100 nM or preferably less than 10 nM or less than 1 nM) for two (or three) different somatostatin receptors (*e.g*., as defined by the receptor binding assay in U.S. Patent No. 7,084,117). Thus, what is meant by an SSTR-5 and SSTR-2 receptor agonist is a receptor agonist which has a higher binding affinity (*i.e*., lower Ki) for SSTR-5 and for SSTR-2 than for other somatostatin receptors, *i.e*., SSTR-1, SSTR-3 or SSTR-4.

In one embodiment, the SSTR-1 agonist is also an SSTR-1 selective agonist and the SSTR-2 agonist is also an SSTR-2 selective agonist

Examples of SSTR-1 agonists which may be used to practice the present invention includes, but is not limited to:
Caeg-c[D-Cys-3-Pal-D-Trp-Lys-D-Cys]-Thr(Bzl)-Tyr-NH₂,
Aaeg-c[D-Cys-Pal-D-Trp-Lys-D-Cys]-Thr(Bzl)-Tyr-NH₂;
Taeg-c[D-Cys-Phe-D-Trp-Lys-D-Cys]-Thr(Bzl)-Tyr-NH₂;
Taeg-c[D-Cys-3-Pal-D-Trp-Lys-D-Cys]-Ser(Bzl)-Tyr-NH₂; and
Caeg-c[D-Cys-Phe-D-Trp-Lys-D-Cys]-Thr(Bzl)-Tyr-NH₂;

Examples of SSTR-2 agonists which may be used to practice the present invention include, but are not limited to:
D-Phe-Cys-Phe-D-Trp-Lys-Thr-Cys-Thr-ol;
Dop2-D-Lys(Dop2)-c[Cys-Tyr-D-Trp-Lys-Abu-Cys]-Thr-NH₂;
D-β-Nal-c[Cys-Tyr-D-Trp-Lys-Val-Cys]-Thr-NH₂;
c[Tic-Tyr-D-Trp-Lys-Abu-Phe];
4-(2-Hydroxyethyl)-1-piperazinylacetyl-D-Phe-c[Cys-Tyr-D-Trp-Lys-Abu-Cys]-Thr-NH₂; and
4-(2-Hydroxyethyl)-1-piperazine-2-ethanesulfonyl-D-Phe-c[Cys-Tyr-D-Trp-Lys-Abu-Cys]-Thr-NH₂.

Examples of SSTR-5 agonists which may be used to practice the present invention include, but are not limited to:
D-Phe-Phe-Trp-D-Trp-Lys-Thr-Phe-Thr-NH₂ and
D-Phe-[Cys-Tyr(I)-D-Trp-Lys-Val-Cys]-Thr-NH₂

An example of an agonist binding to two different SSTR receptors, in this case, SSTR-5 and SSTR-2, which may be used to practice the present invention includes, but is not limited to, D-Phe-[Cys-Tyr(I)-D-Trp-Lys-Val-Cys]-Thr-NH₂.

Further examples of somatostatin agonists are those covered by formulae or those specifically recited in the publications set forth below.
EP Application No. P5 164 EU (Inventor: G. Keri);
Van Binst, G. et al. Peptide Research 5:8 (1992);
Horvath, A. et al. Abstract, "Conformations of Somatostatin Analogs Having Antitumor Activity", 22nd European peptide Symposium, September 13-19, 1992, Interlaken, Switzerland;
PCT Application No. WO 91/09056 (1991);
EP Application No. 0 363 589 A2 (1990);
U.S. Patent No. 4,904,642 (1990);
U.S. Patent No. 4,871,717 (1989);
U.S. Patent No. 4,853,371 (1989);
U.S. Patent No. 4,725,577 (1988);
U.S. Patent No. 4,684,620 (1987);
U.S. Patent No. 4,650,787 (1987);
U.S. Patent No. 4,603,120 (1986);
U.S. Patent No. 4,585,755 (1986);
EP Application No. 0 203 031 A2 (1986);
U.S. Patent No. 4,522,813 (1985);
U.S. Patent No. 4,486,415 (1984);
U.S. Patent No. 4,485,101 (1984);
U.S. Patent No. 4,435,385 (1984);
U.S. Patent No. 4,395,403 (1983);
U.S. Patent No. 4,369,179 (1983);
U.S. Patent No. 4,360,516 (1982);
U.S. Patent No. 4,358,439 (1982);
U.S. Patent No. 4,328,214 (1982);
U.S. Patent No. 4,316,890 (1982);
U.S. Patent No. 4,310,518 (1982);
U.S. Patent No. 4,291,022 (1981);
U.S. Patent No. 4,238,481 (1980);
U.S. Patent No. 4,235,886 (1980);
U.S. Patent No. 4,224,199 (1980);
U.S. Patent No. 4,211,693 (1980);
U.S. Patent No. 4,190,648 (1980);
U.S. Patent No. 4,146,612 (1979);
U.S. Patent No. 4,133,782 (1979);
U.S. Patent No. 5,506,339 (1996);
U.S. Patent No. 4,261,885 (1981);
U.S. Patent No. 4,728,638 (1988);
U.S. Patent No. 4,282,143 (1981);
U.S. Patent No. 4,215,039 (1980);
U.S. Patent No. 4,209,426 (1980);
U.S. Patent No. 4,190,575 (1980);
EP Patent No. 0 389 180 (1990);
EP Application No. 0 505 680 (1982);
EP Application No. 0 083 305 (1982);
EP Application No. 0 030 920 (1980);
PCT Application No. WO 88/05052 (1988);
PCT Application No. WO 90/12811 (1990);
PCT Application No. WO 97/01579 (1997);
PCT Application No. WO 91/18016 (1991);
U.K. Application No. GB 2,095,261 (1981); and
French Application No. FR 2,522,655 (1983).

Note that for all somatostatin agonists described herein, each amino acid residue represents the structure of -NH-C[R)H-CO-, in which R is the side chain (*e.g*., CH₃ for Ala). Lines between amino acid residues represent peptide bonds which join the amino acids. Also, where the amino acid residue is optically active, it is the L-form configuration that is intended unless D-form is expressly designated. For clarity, disulfide bonds (*e.g*., disulfide bridge) which exist between two free thiols of Cys residues are not shown. Abbreviations of the common amino acids are in accordance with IUPAC-IUB recommendations.

Some of the compounds of the disclosure can have at least one asymmetric center. Additional asymmetric centers may be present on the molecule depending upon the nature of the various substituents on the molecule. Each such asymmetric center will produce two optical isomers and it is intended that all such optical isomers, as separated, pure or partially purified optical isomers, racemic mixtures or diastereomeric mixtures thereof, are included within the scope of the instant invention.

The compounds of the disclosure generally can be isolated in the form of their pharmaceutically acceptable acid addition salts, such as the salts derived from using inorganic and organic acids. Examples of such acids are hydrochloric, nitric, sulfuric, phosphoric, formic, acetic, trifluoroacetic, propionic, maleic, succinic, D-tartaric, L-tartaric, malonic, methane sulfonic and the like. In addition, certain compounds containing an acidic function such as a carboxy can be isolated in the form of their inorganic salt in which the counter-ion can be selected from sodium, potassium, lithium, calcium, magnesium and the like, as well as from organic bases.

The pharmaceutically acceptable salts can be formed by taking about 1 equivalent of an SSTR-2 agonist, *e.g.*, c[Tic-Tyr-D-Trp-Lys-Abu-Phe], and contacting it with about 1 equivalent or more of the appropriate corresponding acid of the salt which is desired. Work-up and isolation of the resulting salt is well-known to those of ordinary skill in the art.

The compounds of this disclosure can be administered by oral, parenteral (e.g., intramuscular, intraperitoneal, intravenous or subcutaneous injection, or implant), nasal, vaginal, rectal, sublingual or topical routes of administration and can be formulated with pharmaceutically acceptable carriers to provide dosage forms appropriate for each route of administration. Accordingly, the present application discloses pharmaceutical compositions comprising, as an active ingredient, at least one SSTR-1 agonist and at least one SSTR-2 agonist in association with a pharmaceutically acceptable carrier or a combination of two pharmaceutical compositions wherein the first is comprised of an active ingredient of at least one SSTR-1 agonist in association with a pharmaceutically acceptable carrier and the second is comprised of an active ingredient of at least one SSTR-2 agonist in association with a pharmaceutically acceptable carrier.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups, the elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this disclosure for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as coca butter or a suppository wax.

Compositions for nasal or sublingual administration are also prepared with standard excipients well known in the art.

To best treat those patients, the time to resolution of the symptoms should be rapid and coincide with the suppression of circulating calcitonin levels. Treatment of symptomatic, refractory would be a life time treatment in a patient population with a reasonable life expectancy.

In general, an effective dosage of active ingredient in the compositions of this invention may be varied; however, it is necessary that the amount of the active ingredient be such that a suitable dosage form is obtained. The selected dosage depends upon the desired therapeutic effect, on the route of administration, and on the duration of the treatment, all of which are within the realm of knowledge of one of ordinary skill in the art. Generally, dosage levels of between 0.0001 to 100 mg/kg of body weight daily are administered to humans and other animals, e.g., mammals.

A preferred dosage range is 0.01 to 10.0 mg/kg of body weight daily, which can be administered as a single dose or divided into multiple doses.

### EXAMPLES

### 1) Synthesis of somatostatin agonists

The methods for synthesizing somatostatin agonists are well documented and are within the ability of a person of ordinary skill in the art.

Synthesis of short amino acid sequences is well established in the peptide art. For example, synthesis of H-D-Phe-Phe-Trp-D-Trp-Lys-Thr-Phe-Thr-NH₂, described above, can be achieved by following the protocol set forth in Example I of European Patent Application 0 395 417 A1 (incorporated herein by reference in its entirety). The synthesis of somatostatin agonists with a substituted N-terminus can be achieved, for example, by following the protocol set forth in WO 88/02756 (incorporated herein by reference in its entirety), European Patent Application No. 0 329 295(incorporated herein by reference in its entirety), and PCT Publication No. WO 94/04752 (incorporated herein by reference in its entirety).

### 2) MTC tumor studies

### 2A) Patient and sample Information

Eighteen medullary thyroid carcinoma samples, obtained from donors averaging 55.4 years in age (± 3 years with median 54 years old), were selected on the basis of verification of calcitonin, CgA, SSTR-1, SSTR-2 and SSTR-5 mRNA expression by RT-PCR analysis of an initial group of thirty-six samples obtained from patients diagnosed with medullary thyroid carcinoma which were surgically removed. All surgeries took place at the Section of Endocrinology and at the Section of General Surgery of the University of Ferrara, Ferrara, Italy and at the Department of General Surgery III of the University of Padua, Padua, Italy between 2003 and 2005. Table 1 shows patients' characteristics and pre-operative hormonal values.

**Table 1: Patients' clinical data.**

| Patient N° | Sex | Age | Plasma CT* (pg/ml) |
|---|---|---|---|
| # 1 | F | 75 | 1500 |
| # 2 | M | 42 | 9227 |
| # 3 | M | 71 | 58 |
| # 4 | M | 51 | 3405 |
| # 5 | F | 42 | 3 |
| # 6 | F | 79 | 153 |
| # 7 | M | 39 | 97 |
| # 8 | M | 61 | 1500 |
| # 9 | F | 53 | 306 |
| # 10 | M | 69 | 1500 |
| # 11 | F | 44 | 19 |
| # 12 | F | 38 | 28 |
| # 13 | F | 71 | 48 |
| # 14 | F | 37 | 2350 |
| # 15 | F | 64 | 74 |
| # 16 | F | 27 | 940 |
| # 17 | M | 12 | 1500 |
| # 18 | M | 30 | 700 |

| | | | |
|---|---|---|---|
| *CT = calcitonin | | | |

All patients (8 males and 10 females; age = 50.3 ± 4.6 years old) underwent a total thyroidectomy with central neck lymph node clearance after histological and immunohistochemical diagnosis of medullary thyroid carcinoma. Tissue from the primary tumour was obtained from thirteen patients of the selected patient population while tissue from the metastatic lymph nodes of five different members of the test population was also procured.

### 2B) Preparation of cell cultures

Tissue samples were surgically removed in accordance with the guidelines of the local committees on human research. Thyroid tissue samples were obtained under sterile conditions and immediately minced in RPMI 1640 tissue culture medium. Tissues were incubated with 0.25% trypsin overnight at 4° C and then dissociated using 0.35 % collagenase (Sigma, Milano, ITALY) and 1% trypsin at 37°C for sixty minutes. Cell suspensions were filtered through double layers of gauze and washed twice with serum-free F-12 Ham's Nutrient Modified Medium (F-12; Euroclone Ltd, Wetherby, UNITED KINGDOM). Tumor cells were re-suspended in F-12 with 10% fetal bovine serum and antibiotics, seeded in ninety-six well culture plates (∼2 x 10⁴ cells/well) and incubated at 37°C in a humidified atmosphere of 5% CO₂ and 95% air according to known procedures (Zatelli, M. C. et al., Endocrinology, 2005, 146:2692-8). Approximately twenty-four hours later, the prepared cells were treated with the selected somatostatin agonist and were monitored for hormone secretion and overall viability. Informed consent of the patients was obtained for disclosing clinical investigation and performing the *in vitro* study.

### 2C) Isolation of RNA and RT-PCR

In order to demonstrate the origin of the samples from parafollicular C-cells, RT-PCR analysis for calcitonin expression was performed on each specimen. Further expression analysis for CgA, SSTR-1, SSTR-2, SSTR-3, SSTR-4 and SSTR-5 was performed with only calcitonin expressing tissues as previously described in the art (Zatelli, M. C. et al., J. Clin. Endocrinol. Metab., 2005, 90:2104-9). Frozen tissues were disrupted and total RNA from the pulverized tumours was isolated with TRIZOL® reagent (Invitrogen, Milano, ITALY) according to the manufacturer's protocol.

To prevent DNA contamination, the RNA was treated with RNase-free deoxyribonuclease (Promega, Milano, ITALY). Using a first strand complementary DNA (cDNA) synthesis kit (SuperScript Preamplification System for First Strand cDNA Synthesis®, Invitrogen, Milano, ITALY), 1 µg of total RNA was reverse transcribed with random hexamers according to the manufacturer's protocol. The reverse transcription (RT) reaction was carried out in a GeneAmp 9700 PCR System® (Applera Italia, Monza, ITALY) using previously known protocols (Zatelli, M. C. et al., Horm. Metab. Res., 2003, 35:349-54).

The cDNA (1 µl of RT reaction) was then amplified by PCR with 1 U Taq DNA polymerase (Invitrogen, Milano, ITALY) under conditions recommended by the supplier. Integrity of the cDNA was tested by demonstrating the presence of a GAPDH signal. PCR reactions were carried out using the oligonucleotide primers as detailed in Table 2 and as previously described (Zatelli, M. C. et al., Horm. Metab. Res., 2002, 34:229-33; Zatelli, M. C. et al., J. Clin. Endocrinol. Metab., 2004, 89:5181-8; Zatelli, M. C. et al., J. Clin. Endocrinol. Metab., 2003, 88:2797-802).

**Table 2: Primers and PCR Conditions for GAPDH, Calcitonin, CgA and SSTR Amplification**

| | Primers | Denaturation | Annealing | Extension | # Cycles | Expected fragment (base pairs) |
|---|---|---|---|---|---|---|
| GAPDH | For: 5′ -- ATG ACC CCT TCA TTG ACC TC - 3′ | 95°C, 30 sec | 60°C, 1 min | 72°C, 2 min | 40 | 820 |
| | Rev: 5′ -- AAG TGG TCG TTG AGG GCA AT - 3′ | | | | | |
| CT | For: 5'- GAG AGG TGT CAT GGG CTT CC - 3' | 95°C, 1 min | 62°C, 1 min | 72°C, 1.5 min | 35 | 700 |
| | Rev:5′- GCA CAT TCA GAA GCA GGA CA - 3′ | | | | | |
| CgA | For: 5'- AAC CAG AGC AGC CAG GCC GAG- 3' | 94°C, 1 min | 60°C, 1 min | 72°C, 2 min | 30 | 265 |
| | Rev: 5′ – TGG CTG GAG GGT GGG TGT TGG- 3′ | | | | | |
| SSTR1 | For: 5' – AGC CGG TTG ACT ATT ACG CC - 3' | 95°C, 45 sec | 59°C, 1 min | 74°C, 40 sec | 45 | 334 |
| | Rev: 5'- GCT CTC ACT TCT ACC ATT GTC - 3' | | | | | |
| SSTR 2 | For: 5' – CGC AGA TGA GCT CTT CAT GCT-3' | 95°C, 45 sec | 58°C, 45 sec | 74°C, 45 sec | 35 | 158 |
| | Rev: 5′ – GGT CGA TGC TCA TGA CTG TCA - 3′ | | | | | |
| SSTR 3 | For: 5′ – CTG GAT GCC CTT CTA CGT GCT - 3′ | 95°C, 30 sec | 60°C, 45 sec | 74°C, 45 sec | 35 | 152 |
| | Rev: 5' – GAG CCC AAA GAA GGC AGG CT - 3' | | | | | |
| SSTR4 | For: 5′- GCG CTC GGA GAA GAA AAT CAC - 3′ | 94°C, 30 sec | 58°C, 30 sec | 72°C, 30 sec | 45 | 201 |
| | Rev: 5′ – AAG TTG TCG GAG AGG AAG CCA - 3′ | | | | | |
| SSTR5 | For: 5'- TCT TCA CCG TCA ACA TCG TCA- 3' | 94°C, 45 sec | 57°C, 45 sec | 74°C, 45 sec | 35 | 154 |
| | Rev: 5′ – AAG CTC TGG CGG AAG TTG TCA - 3′ | | | | | |

### 2D) Quantitative PCR for SSTR-1, SSTR-2 and SSTR-5 mRNA

Using the human endogenous control plate (Applera Italia, Monza, ITALY) according to the manufacturer's instructions, an endogenous control gene was selected. Total RNA was isolated as described above from three medullary thyroid carcinoma samples and from a pool of three samples of normal thyroid tissue deriving from the same patient. The candidate genes included 18S rRNA, acidic ribosomal protein, β-action, cyclophilin, GAPDH, phosphoglycerokinase, β-microglobulin, human β-glucuronidase (huGUS), hypoxanthine ribosyl transferase, transcription factor IID TATA-binding protein, and transferrin receptor. With the exception of 18S rRNA, the primers and the probes of the candidate genes recognized only cDNA. All measurements were performed in duplicate experiments. The results are expressed as "threshold cycle value (ΔCT)" representing the difference between the threshold cycle obtained of the normal thyroid pool and the threshold cycle recorded for the samples for each housekeeping gene. The normal thyroid pool value serves as a baseline for the assays and is considered as zero.

Quantitative PCR for SSTR-1, SSTR-2 and SSTR-5 was performed using procedures previously disclosed (Zatelli, M. C. et al., Endocrinology, 2005, 146:2692-8; Zatelli, M. C. et al., . J. Clin. Endocrinol. Metab., 2003, 88:2797-802) with primers and probes designed using Primer Express Software ^{®} (Applera Italia, Monza, ITALY). TaqMan ^{®} probes (Applera Italia, Monza, ITALY) labelled with a fluorescent dye (6-carboxy-fluorescein, FAM) and a quencher dye (6-carboxy-tetramethyl rhodamine, TAMRA) were used. Pre-Developed TaqMan ^{®} Assay Reagents for huGUS (20x) were used to asses retrotranscription efficiency (Applera Italia, Monza, ITALY).

Serial dilutions of the single stranded SSTR-1, SSTR-2 and SSTR-5 sense oligonucleotide amplicons (from 10⁹ to 10² molecules) were carried out in triplicate. The log copy numbers of unknown samples were calculated from the regression line according to the equation: logN=(Ct-q)/m, where Ct is the threshold cycle, q is the γ-intercept, and m is the slope of the standard curve line. All Quantitative PCR reactions were performed, recorded and analysed using the ABI 7700 Prism Sequence Detection System ^{®} (Applera Italia, Monza, ITALY). Slopes for all assays reported were -3.3 ± 0.1.

All samples were carried out in triplicate (100 ng of reverse transcribed total RNA per well) and repeated at least twice. No Template Control and RT- controls were run in each experiment.

A cut-off of 3 x 10³ mRNA copies/µg total RNA was established as the threshold for Real Time PCR to exclude the detection of transcripts due to illegitimate transcription, as previously suggested (Korbonits, M. et al., J. Clin. Endocrinol. Metab., 2001, 86:881-7; and Chelly, J. et al., Proc. Natl. Acad. Sci. U S A, 2001, 86:2617-21).

### 2E) Somatostatin and SSTR Selective Agonists

Somatostatin analogs (IPSEN (Biomeasure, Inc.), Milford, Massachusetts, UNITED STATES OF AMERICA) used in this study and their respective affinities to the different SSTR are listed below in Table 3:

**Table 3: SSTR Specificity Ki**

| | **SSTR1** | **SSTR2** | **SSTR3** | **SSTR4** | **SSTR5** |
|---|---|---|---|---|---|
| **COMPOUND** | | | | | |
| lanreotide D-β-Nal-c[Cys-Tyr-D-Trp-Lys-Val- Cys]-Thr-NH2 | 2129 | 0.75 | 98 | 1826 | 12.7 |
| Aaeg-[D-Cys-Pal-D-Trp-Lys-D-Cys]-Thr(Bzl)- Tyr-NH2 | 3.7 | >1000 | >1000 | >1000 | 458 |
| Caeg-c[D-Cys-3-Pal-D-Trp-Lys-D-Cys]- Thr(Bzl)-Tyr-NH2 | 4.33 | >1000 | >1000 | 833 | 606 |
| c[Tic-Tyr-D-Trp-Lys-Abu-Phe] | 1000 | 0.34 | 412 | 1000 | 213.5 |
| D-Phe-Phe- Trp-D- Trp-Lvs-Thr-Phe- Thr-NH₂ | 1152 | 166 | 1000 | 1618 | 2.4 |
| D-Phe-[Cys-Tyr(I)-D-Trp-Lys-Val-Cys]-Thr- NH2 | 1020 | 0.29 | 133 | >1000 | 0.67 |
| Dop2-D-Lys(Dop2)-c[Cys-Tyr-D-Trp-Lys- Abu-Cys]-Thr-NH2 | 673.8 | 0.14 | 99.2 | >1000 | 41.5 |
| 4-(2-Hydroxyethyl)-1-piperazinylacetyl-D- Phe-c[Cys-Tyr-D-Trp-Lys-Abu-Cys]-Thr-NH2 | 4516 | 0.30 | 214.6 | 6358 | 10.6 |
| 4-(2-Hydroxyethyl)-1-piperazine-2- ethanesulfonyl-D-Phe-c[Cys-Tyr-D-Trp-Lys- Abu-Cys]-Thr-NH2 | 6016 | 0.18 | 26.8 | 3897 | 9.81 |
| Taeg-c[D-Cys-Phe-D-Trp-Lys-D-Cys]- Thr(Bzl)-Tyr-NH2 | 8.48 | >1000 | >1000 | 530 | 72.8 |
| Taeg-c[D-Cys-3-Pal-D-Trp-Lys-D-Cys]- Ser(Bzl)-Tyr-NH2 | 3.74 | >1000 | >1000 | 277 | 211 |
| Caeg-c[D-Cys-Phe-D-Trp-Lys-D-Cys]- Thr(Bzl)-Tyr-NH2 | 2.81 | >1000 | >1000 | 918 | 150 |

### 2F) Cell Viability

The effects of the tested somatostatin analogs on medullary thyroid carcinoma primary culture cell viability were assessed with a Cell Proliferation Kit I MTT ® (Roche Diagnostics GmbH, Mannheim, GERMANY) which employs a colorimetric method for determining the number of viable cells in proliferation assays. Primary cultured cells were plated in ninety-six multiwell plates (2 x 10⁴ cells/well) and incubated for forty-eight hours in a medium supplemented with 10% fetal bovine serum in the presence or absence of each somatostatin analog at 10⁻⁸ M. Treatments were renewed after the first twenty-four hours of incubation. The absorbance at 560 nm was then recorded using microplate reader (Victor, Perkin Elmer, Monza, ITALY). Results (absorbance at 560 nm) were obtained by determining the mean value of at least six experiments in eight replicates using known procedures previously described (Zatelli, M. C. et al., Biochem. Biophys. Res. Commun., 2002, 297:828-34).

### 2G) Calcitonin Assay

Calcitonin levels were measured in a conditioned medium from treated and untreated primary cultured cells with the calcitonin-U.S.-IRMA^{®} kit (Biosource Europe, Nivelles, BELGIUM) after a six hour treatment with 10⁻⁸ M of the selected somatostatin analogs. The intra- and inter-assay variation coefficients were 1.9- 2.7 % and 1.9 - 3.3 %, respectively. The detection limit was 0.8 pg/ml. Hormone assays were performed in duplicate of conditioned medium from treated cells. Results were obtained by determining the mean value among eight replicates. Primary cultures were considered as "responders" when a significant reduction >15% in calcitonin secretion was recorded under treatment with lanreotide.

### 2H) CgA Secretion

The effects of the selected somatostatin analogs on CgA secretion were analysed by measuring human CgA immunoreactivity in the culture medium from primary cultured cells incubated for six hours with or without 10⁻⁸ M each somatostatin analog using an ELISA kit (Dako, Milano, ITALY), according to the process previously described (Zatelli, M. C. et al., Horm. Metab. Res., 2003, 35:349-54). The detection limit was 2.0 U/L, with intra- and inter-assay coefficients of variation of 5.8% and 8.6%, respectively. Hormone assays were performed in duplicate. Results were obtained by determining the mean value among eight replicates.

### 2I Statistical Analysis

Data are expressed as the mean ± standard error (SE). A preliminary analysis was carried out to determine whether the datasets conformed to a normal distribution and a computation of homogeneity of variance was performed using Bartlett's test. The results were compared within each group and between groups using ANOVA. If the F values were significant (P< 0.05), then either Student's paired or unpaired t test was used to evaluate the individual differences between means. To measure the strength of association between pairs of variables without specifying dependencies Spearman order correlations were run. A P<0.05 was considered significant in all tests.

### 3) Results and discussion of SSTR expression patterns in medullary thyroid carcinoma

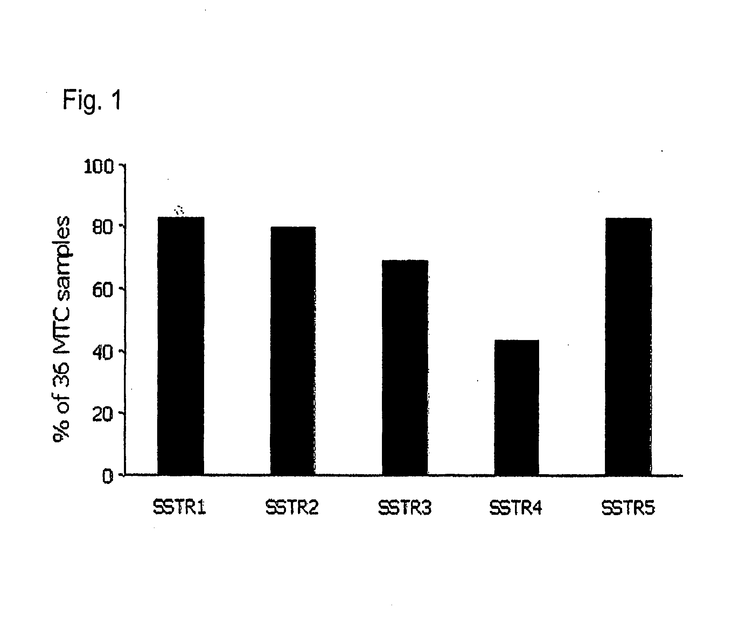

Thirty-six surgically removed medullary thyroid carcinoma samples underwent RT-PCR analysis for calcitonin, CgA, and SSTR1-5 mRNA expression and eighteen tumours (50%) were selected on the basis of the expression of calcitonin, CgA, SSTR-1, SSTR-2 and SSTR-5 mRNAs (see Table 4; see also Figure 1):

**Table 4: Primary cultures in vitro hormone secretion and SSTR expression pattern**

| | **In vitro levels** | | **Expression pattern** | | | | | | |
|---|---|---|---|---|---|---|---|---|---|
| N° | CT (pg/ml) | CgA (U/l) | CT | CgA | SSTR1 | SSTR2 | SSTR3 | SSTR4 | SSTR5 |
| # 1 | 383.2 | 23.4 | + | + | + | + | + | + | + |
| # 2 | 34.4 | 3.2 | + | + | + | + | - | - | + |
| # 3 | 136.4 | 12.1 | + | + | + | + | + | - | + |
| # 4 | 770.8 | 7.9 | + | + | + | + | + | - | + |
| # 5 | 161.5 | 8.2 | + | + | + | + | + | - | + |
| # 6 | 1276.5 | 8.3 | + | - | + | + | - | - | + |
| # 7 | 2418.4 | 32.4 | + | + | + | + | + | + | + |
| # 8 | 1569.2 | 15.6 | + | + | + | + | + | + | + |
| # 9 | 892.5 | 25.6 | + | + | + | + | + | - | + |
| # 10 | 2500.7 | 40.6 | + | + | + | + | + | + | + |
| # 11 | 730.7 | 7.9 | + | - | + | + | + | + | + |
| #12 | 82.2 | 5.3 | + | + | + | + | - | + | + |
| # 13 | 3866.6 | 8.1 | + | + | + | + | + | + | + |
| # 14 | 2540.2 | 7.6 | + | + | + | + | - | + | + |
| # 15 | 1045.9 | 87 | + | - | + | + | + | + | + |
| # 16 | 1614.6 | 46.5 | + | + | + | + | + | + | + |
| # 17 | 21.8 | 1.6 | + | + | + | + | - | - | + |
| # 18 | 1078.8 | 30.8 | + | + | + | + | + | - | + |

| | | | | | | | | | |
|---|---|---|---|---|---|---|---|---|---|
| Basal secretion of CT and CgA in control culture medium from 12 x 10⁴ cells cultured for 12 h. | | | | | | | | | |

The absolute SSTR mRNA levels were also investigated in the selected samples. By employing the human endogenous control plate, it was learned that the best endogenous control gene, expressed at nearly constant levels in all samples examined, was the huGUS. The assays produced Δcalcitonin values for huGUS slightly different from zero in all examined samples indicating a fairly stable level of gene expression in the samples as compared to the normal thyroid pool. Quantitative PCR showed SSTR-1 mRNA levels of 6.8 x 10⁵ copies/µg total RNA, SSTR-2 of 7.3 x 10⁷ molecules/µg total RNA, and SSTR-5 of 8.5 x 10⁴ copies/µg total RNA. SSTR mRNA expression levels are displayed in Tables 5 and Tables 6.

**Table 5. SSTR mRNA expression levels and percent CT secretion inhibition in the selected 18 MTC primary cultures undergoing treatment with 10⁻⁸ M SRIH analogs**

| | n° copies x 1 µg total RNA | | | % CT secretion inhibition vs. control | | | | | |
|---|---|---|---|---|---|---|---|---|---|
| N° | SSTR1 | SSTR2 | SSTR5 | Lan | Cmpd #1 | Cmpd # 2 | Cmpd # 3 | Cmpd # 4 | Cmpd # 1 + #2 |
| #1^{†} | 1.5 x 10⁵ | 3.3 x 10⁷ | 1.5 x 10⁴ | -5.2 | -7.7 | -6.1 | -16.6 | -10.5 | 15.2 |
| #2^{†} | 2.9 x 10⁴ | 1.6 x 10⁷ | 4.9 x 10³ | 21.7 | -13.8 | -4.3 | 18.2 | 5.2 | -10.6 |
| #3^{§} | 1.9 x 104 | 1.4 x 10⁷ | 1.8 x 10⁴ | -22.6 | -15.6 | -36.0 | -36.5 | -33.9 | -15.8 |
| #4^{†} | 6.4 x 10⁴ | 7.2 x 10⁷ | 2.2 x 10⁴ | 34.7 | -20.6 | 8.9 | 5.3 | -10.3 | 6.3 |
| #5^{§} | 6.2 x 10³ | 2.5 x 10⁷ | 3.2 x 10³ | -19.0 | -15.1 | -8.6 | -17.5 | -15.7 | -9.9 |
| #6^{§} | 1.3 x 106 | 9.5 x 10⁶ | 5.8 x 10⁵ | -18.0 | -20.2 | -5.7 | -25.8 | -12.6 | -12.7 |
| #7^{§} | 9.0 x 10⁵ | 2.2 x 10⁸ | 5.9 x 10³ | -44.6 | -10.5 | -51.3 | -34.6 | -42.6 | -62.6 |
| #8^{†} | 1.1 x 10⁵ | 2.4 x 10⁷ | 2.8 x 10⁴ | 14.7 | -12.8 | -2.6 | -7.5 | 3.6 | 7.1 |
| #9^{†} | 5.7 x 10⁵ | 6.3 x 10⁷ | 7.9 x 10³ | 9.8 | -9.4 | 6.7 | -1.6 | -5.2 | -7.7 |
| #10^{†} | 1.8 x 10⁴ | 2.1 x 10⁷ | 1.5 x 10⁴ | 22.9 | -3.8 | 11.6 | 15.6 | 12.8 | -2.7 |
| #11^{§} | 7.4 x 10⁴ | 7.2 x 10⁷ | 3.2 x 10⁴ | -25.1 | -25.8 | 10.3 | -76.2 | -15.7 | -75.9 |
| #12^{§} | 5.8 x 10⁵ | 5.8 x 10⁷ | 2.4 x 10⁴ | -20.6 | -16.8 | -12.4 | -9.3 | -18.5 | -34.6 |
| #13^{§} | 1.7 x 10⁶ | 2.5 x 10⁸ | 8.7 x 10³ | -42.5 | -13.3 | -48.6 | -43.2 | -42.8 | -66.4 |
| #14^{§} | 1.6 x 10⁶ | 1.3 x 10⁸ | 3.3 x 10⁴ | -19.8 | -12.1 | 1.6 | 9.5 | 8.4 | -8.3 |
| #15^{§} | 3.5 x 10⁶ | 9.7 x 10⁷ | 6.9 x 10⁵ | -20.9 | -22.6 | -22.9 | 7.8 | -30.6 | -21.4 |
| #16^{§} | 1.0 x 10⁵ | 1.3 x 10⁸ | 1.2 x 10⁴ | -34.3 | -14.7 | -31.7 | -17.4 | -16.2 | -40.7 |
| #17^{†} | 8.5 x 10⁵ | 4.3 x 10⁷ | 9.4 x 10³ | 8.5 | -26.9 | -2.6 | 7.4 | 12.7 | -9.6 |
| #18^{†} | 6.8 x 10⁵ | 4.4 x 10⁷ | 1.6 x 10⁴ | -10.8 | -14.6 | -1.5 | 13.6 | 5.3 | -12.1 |

| | | | | | | | | | |
|---|---|---|---|---|---|---|---|---|---|
| §Responder and †non responder MTC primary cultures | | | | | | | | | |

The compounds represented in Table 5 are as follows:
Compound #1: Caeg-c[D-Cys-3-Pal-D-Trp-Lys-D-Cys]-Thr(Bzl)-Tyr-NH₂
Compound #2: c[Tic-Tyr-D-Trp-Lys-Abu-Phe]
Compound #3: D-Phe-Phe-Trp-D-Trp-Lys-Thr-Phe-Thr-NH₂
Compound #4: D-Phe-[Cys-Tyr(I)-D-Trp-Lys-Val-Cys]-Thr-NH₂

**Table 6. SSTR mRNA expression levels and percent cell viability reduction in the selected 18 MTC primary cultures undergoing treatment with 10⁻⁸ M SRIH analogs**

| | n° copies x 1 eg total DNA | | | % cell viability reduction vs. control | | | | | |
|---|---|---|---|---|---|---|---|---|---|
| N° | SSTR1 | SSTR2 | SSTR5 | Lan | Cmpd #1 | Cmpd #2 | Cmpd #3 | Cmpd #4 | Cmpd #1 + #2 |
| #1^{†} | 1.5 x 10⁵ | 3.3 x 10⁷ | 1.5 x 10⁴ | -6.1 | 66.6 | -5.0 | 30.8 | -14.7 | -10.8 |
| #2^{†} | 2.9 x 10⁴ | 1.6 x 10⁷ | 4.9 x 10³ | -57.0 | -73.8 | -56.4 | -72.5 | -38.3 | -25.8 |
| #3^{§} | 1.9 x 10⁴ | 1.4 x 10⁷ | 1.8 x 10⁴ | 1.5 | -1.7 | -3.2 | 0.4 | 13.3 | 4.7 |
| #4^{†} | 6.4 x 10⁴ | 7.2 x 10⁷ | 2.2 x 10⁴ | -25.8 | -5.7 | -17.9 | 4.8 | -11.3 | 15.6 |
| #5^{§} | 6.2 x 10³ | 2.5 x 10⁷ | 3.2 x 10³ | 13.4 | -10.3 | -8.6 | -5.3 | 24.7 | 21.7 |
| #6^{§} | 1.3 x 10⁶ | 9.5 x 10⁶ | 5.8 x 10⁵ | 9.7 | 21.7 | 3.2 | 2.4 | -10.8 | -5.8 |
| #7^{§} | 9.0 x 10⁵ | 2.2 x 10⁸ | 5.9 x 10³ | 54.4 | 14.2 | 54.4 | 4.2 | 5.7 | 8.2 |
| #8^{†} | 1.1 x 10⁵ | 2.4 x 10⁷ | 2.8 x 10⁴ | -7.9 | 7.4 | -15.3 | -0.3 | -20.6 | 9.7 |
| #9^{†} | 5.7 x 10⁵ | 6.3 x 10⁷ | 7.9 x 10³ | -8.6 | 13.7 | -8.8 | 3.6 | -32.1 | 16.4 |
| #10^{†} | 1.8 x 10⁴ | 2.1 x 10⁷ | 1.5 x 10⁴ | 7.0 | 3.5 | 12.1 | 2.3 | 6.5 | 2.5 |
| #11^{§} | 7.4 x 10⁴ | 7.2 x 10⁷ | 3.2 x 10⁴ | -11.2 | -4.1 | -0.4 | -1.0 | 1.4 | 12.4 |
| #12^{§} | 5.8 x 10⁵ | 5.8 x 10⁷ | 2.4 x 10⁴ | 3.5 | -5.4 | 15.8 | -11.3 | 7.4 | 7.3 |
| #13^{§} | 1.7 x 10⁶ | 2.5 x 10⁸ | 8.7 x 10³ | 23.1 | -11.8 | -5.3 | 7.2 | 9.3 | -1.3 |
| #14^{§} | 1.6 x 10⁶ | 1.3 x 10⁸ | 3.3 x 10⁴ | 1.8 | 7.3 | 8.2 | 6.0 | 1.3 | 8.6 |
| #15^{§} | 3.5 x 10⁶ | 9.7 x 10⁷ | 6.9 x 10⁵ | -8.3 | 6.1 | 5.6 | 5.2 | 12.6 | -2.6 |
| #16^{§} | 1.0 x 10⁵ | 1.3 x 10⁸ | 1.2 x 10⁴ | -15.8 | -0.3 | -3.0 | -5.8 | 15.0 | 0.3 |
| #17^{†} | 8.5 x 10⁵ | 4.3 x 10⁷ | 9.4 x 10³ | -9.7 | -9.7 | -10.3 | -8.7 | -2.6 | 8.5 |
| #18^{†} | 6.8 × 10⁵ | 4.4 x 10⁷ | 1.6 x 10⁴ | -15.8 | -2.6 | -20.6 | -3.8 | -7.7 | 5.7 |

| | | | | | | | | | |
|---|---|---|---|---|---|---|---|---|---|
| §Responder and †non responder MTC primary cultures | | | | | | | | | |

The compounds represented in Table 6 are as follows:
Compound #1: Caeg-c[D-Cys-3-Pal-D-Trp-Lys-D-Cys]-Thr(Bzl)-Tyr-NH₂
Compound #2: c[Tic-Tyr-D-Trp-Lys-Abu-Phe]
Compound #3: D-Phe-Phe-Trp-D-Trp-Lys-Thr-Phe-Thr-NH₂
Compound #4: D-Phe-[Cys-Tyr(I)-D-Trp-Lys-Val-Cys]-Thr-NH₂

SSTR-2 mRNA expression levels were significantly (P<0.01) higher than both SSTR-1 and SSTR-5 mRNA expression levels.

### 3B) Effects of somatostatin analogs on calcitonin and CgA secretion by medullary thyroid carcinoma primary cultures

In order to investigate the effects of somatostatin analogs on hormone secretion, calcitonin and CgA levels were measured in conditioned media collected from primary cultures derived from the selected eighteen medullary thyroid carcinoma treated with or without each somatostatin analog at 10⁻⁸M for six hours. As indicated in Table 4, measurable amounts of calcitonin and CgA were detected in the culture medium from all cultured tumour tissues. Percent calcitonin secretion reduction compared with control in each primary culture under treatment with each somatostatin analog is displayed in Table 5.

The primary cultures were then divided in two groups according to the extent of calcitonin secretion inhibition recorded after treatment with D-β-Nal-[Cys-Tyr-D-Trp-Lys-Val-Cys]-Thr-NH₂ (sold as lanreotide). Cultures responding to lanreotide with a calcitonin reduction of 15% or greater when compared to untreated cells were considered "responders". According to this criterion, nine cultures were considered as "responders" (group A: primary cultures #3, #5, #6, #7, #11, #12, #13, #14, #15, #16) and nine as "non responders" (group B: primary cultures #1, #2, #4, #8, #9, #10, #17, #18). SSTR mRNA levels did not significantly differ between the two groups. Group A displayed SSTR-1 mRNA levels of 9.1 x 10⁵ copies/µg total RNA, SSTR-2 of 9.7 x 10⁷ copies/µg total RNA and SSTR-5 of 1.5 x 10⁵ copies/µg total RNA. Group B displayed SSTR-1 mRNA levels of 4.6 x 10⁵ copies/µg total RNA, SSTR-2 of 4.9 x 10⁷ copies/µg total RNA and SSTR-5 of 1.7 x 10⁴ copies/µg total RNA. On the other hand, mean pre-operative calcitonin plasma levels were significantly lower in patients belonging to group A than in patients belonging to group B (158 ± 105 pg/ml vs. 2383 ± 963 pg/ml; P<0.01).

In group A, calcitonin secretion was reduced by 27% after treatment with lanreotide (Fig. 2). Moreover, a direct correlation was evident between SSTR-2 expression levels and lanreotide inhibitory effects on calcitonin secretion (r²= 0.88; P<0.05; see data in Table5). In this group, treatment with the SSTR-1 selective agonist, Caeg-c[D-Cys-3-Pal-D-Trp-Lys-D-Cys]-Thr(Bzl)-Tyr-NH₂ significantly reduced calcitonin secretion (-17%; P<0.05), but the inhibitory effect did not correlate with SSTR-1 expression levels. On the other hand, the inhibitory effect of the SSTR-2 selective agonist, c[Tic-Tyr-D-Trp-Lys-Abu-Phe], on calcitonin secretion (-23%; P<0.05) directly correlated with SSTR-2 expression levels (r²= 0.5; P<0.05) (Fig.5). Treatment with the SSTR-5 selective agonist, D-Phe-Phe-Trp-D-Trp-Lys-Thr-Phe-Thr-NH₂, inhibited calcitonin secretion by 28% (P<0.05), and the inhibitory effect did not correlate with SSTR-5 expression. The dual SSTR-2/SSTR-5 agonist, D-Phe-[Cys-Tyr(I)-D-Trp-Lys-Val-Cys]-Thr-NH₂, significantly reduced calcitonin secretion (-25%; P<0.05) and this effect directly correlated with SSTR-2 expression (r²= 0.7; P<0.05). Finally, treatment with both the SSTR-1 and the SSTR-2 selective agonists {Caeg-c[D-Cys-3-Pal-D-Trp-Lys-D-Cys]-Thr(Bzl)-Tyr-NH₂ + c[Tic-Tyr-D-Trp-Lys-Abu-Phe]} had an additive effect which significantly reduced calcitonin secretion by 38% (P<0.01). The extent of the inhibitory effect of the two compounds directly correlated with SSTR-2 expression levels (r²= 0.67; P<0.05).

In group B, which did not respond to lanreotide, it was discovered that calcitonin secretion was not reduced by treatment with somatostatin analogs interacting with SSTR-2, SSTR-5 or both (c[Tic-Tyr-D-Trp-Lys-Abu-Phe], D-Phe-Phe-Trp-D-Trp-Lys-Thr-Phe-Thr-NH₂, D-Phe-[Cys-Tyr(I)-D-Trp-Lys-Val-Cys]-Thr-NH₂). On the other hand, the SSTR-1 selective agonist, Caeg-c[D-Cys-3-Pal-D-Trp-Lys-D-Cys]-Thr(Bzl)-Tyr-NH₂, significantly reduced calcitonin secretion (-13%; P<0.05) (Fig.3) which did not correlate with SSTR-1 expression levels.

With regard to CgA secretion, the somatostatin analogs did not significantly affect CgA secretion in medullary thyroid carcinoma primary cultures, in either group A or group B, except for medullary thyroid carcinoma # 8. In this sample, belonging to the "non responder" group expressing all SSTR receptors, a significant reduction in CgA secretion after treatment with each SSTR selective agonist alone or in combination (ranging from -35% to -67% CgA secretion vs. control; P<0.05) was noted.

### 3C) Effects of somatostatin analogs on medullary thyroid carcinoma primary cultures cell viability

In order to investigate the effects of somatostatin analogs on medullary thyroid carcinoma cell viability, an MTT assay was performed in primary cultures derived from the eighteen selected medullary thyroid carcinoma samples, treated with or without each Somatostatin analog at 10⁻⁸M for forty-eight hours. Percent cell viability reduction compared to a control in each primary culture under treatment with each somatostatin analog is reported in Table 6.

In general, cellular viability of cells from primary MTC cultures designated as responsive to lanreotide (Group A) was not reduced by treatment with any particular somatostatin analog. In contrast, cellular proliferation of cells from primary MTC cultures designated as non-responsive to lanreotide (Group B) was reduced following treatment with lanreotide (-14%; P<0.01), the SSTR-2 selective agonist c[Tic-Tyr-D-Trp-Lys-Abu-Phe] (-13%; P<0.05) and the dual SSTR-2/SSTR-5 selective agonist, D-Phe-[Cys-Tyr(I)-D-Trp-Lys-Val-Cys]-Thr-NH₂ (-13%; P<0.05). Medullary thyroid carcinoma cell viability was neither affected by treatment with the SSTR-1 selective agonist, Caeg-c[D-Cys-3-Pal-D-Trp-Lys-D-Cys]-Thr(Bzl)-Tyr-NH₂ nor the SSTR-5 selective agonist, D-Phe-Phe-Trp-D-Trp-Lys-Thr-Phe-Thr-NH₂. Moreover, the combination of the SSTR-1 and the SSTR-2 selective agonists did not compromise primary culture cell viability.

## Claims

1. A method of determining the treatment for a patient suffering from medullary thyroid carcinoma comprising the steps of:
a) administering a somatostatin agonist or a pharmaceutically acceptable salt thereof to a sample of said medullary thyroid carcinoma obtained from said patient;
b) measuring the change in calcitonin secreted by said sample after said administration; and
c) determining a treatment,
wherein said determining of said treatment is based upon said identified change in calcitonin secretion.

2. The method according to claim 1, wherein said somatostatin agonist is an SSTR-2 agonist, a selective SSTR-2 agonist or a pharmaceutically acceptable salt thereof.

3. The method according to claim 1, wherein said selection of treatment is based upon a decrease in calcitonin secretion of less than about 15% or a decrease in calcitonin secretion of greater than about 15%.

4. A method according to claim 1 wherein said medullary thyroid carcinoma patient has undergone at least one thyroidectomy.

5. A method according to claim 2, wherein said SSTR-2 agonist is a compound selected from the group consisting of:
D-β-Nal-c[Cys-Tyr-D-Trp-Lys-Val-Cys]-Thr-NH₂;
D-Phe-Cys-Phe-D-Trp-Lys-Thr-Cys-Thr-ol;
Dop2-D-Lys(Dop2)-c[Cys-Tyr-D-Trp-Lys-Abu-Cys]-Thr-NH₂;
c[Tic-Tyr-D-Trp-Lys-Abu-Phe];
4-(2-Hydroxyethyl)-1-piperazinylacetyl-D-Phe-c[Cys-Tyr-D-Trp-Lys-Abu-Cys]-Thr-NH₂; and
4-(2-Hydroxyethyl)-1-piperazine-2-ethanesulfonyl-D-Phe-c[Cys-Tyr-D-Trp-Lys-Abu-Cys]-Thr-NH₂;
or a pharmaceutically acceptable salt thereof.

6. A method according to claim 2, wherein said SSTR-2 agonist compound is D-β-Nal-c[Cys-Tyr-D-Trp-Lys-Val-Cys]-Thr-NH₂ or a pharmaceutically acceptable salt thereof.

7. A method according to claim 2, wherein said SSTR-2 agonist compound D-Phe-Cys-Phe-D-Trp-Lys-Thr-Cys-Thr-ol or a pharmaceutically acceptable salt thereof.

## Patentansprüche

1. Verfahren zum Bestimmen der Behandlung eines Patienten, der unter medullärem Schilddrüsenkarzinom leidet, umfassend die Schritte:
a) Zugeben eines Somatostatin-Agonisten oder eines pharmazeutisch annehmbaren Salzes davon zu einer Probe des medullären Schilddrüsenkarzinoms, erhalten von dem Patienten;
b) Messen der Änderung in der Calcitoninsekretion durch die Probe nach der Zugabe; und
c) Bestimmen einer Behandlung, worin das Bestimmen der Behandlung auf der identifizierten Änderung in der Calcitoninsekretion beruht.

2. Verfahren nach Anspruch 1, worin der Somatostatin-Agonist ein SSTR-2-Agonist, ein selektiver SSTR-2-Agonist oder ein pharmazeutisch annehmbares Salz davon ist.

3. Verfahren nach Anspruch 1, worin die Auswahl der Behandlung auf einer Abnahme in der Calcitoninsekretion von weniger als 15% oder einer Abnahme in der Calcitoninsekretion von mehr als 15% beruht.

4. Verfahren nach Anspruch 1, worin der medulläre Schilddrüsenkarzinom-Patient mindestens eine Schilddrüsenektomie hinter sich hat.

5. Verfahren nach Anspruch 2, worin der SSTR-2-Agonist eine Verbindung ist, ausgewählt aus der Gruppe bestehend aus:
D-β-Nal-c[Cys-Tyr-D-Trp-Lys-Val-Cys]-Thr-NH₂;
D-Phe-Cys-Phe-D-Trp-Lys-Thr-Cys-Thr-ol;
Dop2-D-Lys(Dop2)-c[Cys-Tyr-D-Trp-Lys-Abu-Cys]-Thr-NH₂;
c[Tic-Tyr-D-Trp-Lys-Abu-Phe];
4-(2-Hydroxyethyl)-1-piperazinylacetyl-D-Phe-c[Cys-Tyr-D-Trp-Lys-Abu-Cys]-Thr-NH₂; und
4-(2-Hydroxyethyl)-1-piperazine-2-ethanesulfonyl-D-Phe-c[Cys-Tyr-D-Trp-Lys-Abu-Cys]-Thr-NH₂;
oder ein pharmazeutisch annehmbares Salz davon.

6. Verfahren nach Anspruch 2, worin die SSTR-2-Agonist-Verbindung D-β-Nal-c[Cys-Tyr-D-Trp-Lys-Val-Cys]-Thr-NH₂ oder ein pharmazeutisch annehmbares Salz davon ist.

7. Verfahren nach Anspruch 2, worin die SSTR-2-Agonist-Verbindung D-Phe-Cys-Phe-D-Trp-Lys-Thr-Cys-Thr-ol oder ein pharmazeutisch annehmbares Salz davon ist.

## Revendications

1. Procédé de détermination du traitement pour un patient souffrant d'un carcinome médullaire de la thyroïde, comprenant les étapes consistant à :
a) administrer un agoniste de la somatostatine ou un sel pharmaceutiquement acceptable de celui-ci à un échantillon dudit carcinome médullaire de la thyroïde obtenu auprès dudit patient ;
b) mesurer le changement en calcitonine sécrétée par ledit échantillon après ladite administration ; et
c) déterminer un traitement,
ladite détermination dudit traitement étant basée sur ledit changement identifié en sécrétion de calcitonine.

2. Procédé selon la revendication 1, dans lequel ledit agoniste de la somatostatine est un agoniste de SSTR-2, un agoniste de SSTR-2 sélectif ou un sel pharmaceutiquement acceptable de ceux-ci.

3. Procédé selon la revendication 1, dans lequel ladite sélection de traitement est basée sur une diminution en sécrétion de calcitonine de moins d'environ 15 % ou une diminution en sécrétion de calcitonine de plus d'environ 15 %.

4. Procédé selon la revendication 1, dans lequel ledit patient présentant un carcinome médullaire de la thyroïde a subi au moins une thyroïdectomie.

5. Procédé selon la revendication 2, dans lequel ledit agoniste de SSTR-2 est un composé choisi dans le groupe consistant en :
D-β-Nal-c[Cys-Tyr-D-Trp-Lys-Val-Cys]-Thr-NH₂ ;
D-Phe-Cys-Phe-D-Trp-Lys-Thr-Cys-Thr-ol ;
Dop2-D-Lys(Dop2)-c[Cys-Tyr-D-Trp-Lys-Abu-Cys]-Thr-NH₂;
c[Tic-Tyr-D-Trp-Lys-Abu-Phe] ;
4-(2-hydroxy'thyl)-1-piperazinylacetyl-D-Phe-c[Cys-Tyr-D-Trp-Lys-Abu-Cys]-Thr-NH₂; et
4-(2-hydroxyéthyl)-1-piperazine-2-ethanesulfonyl-D-Phe-c[Cys-Tyr-D-Trp-Lys-Abu-Cys]-Thr-NH₂;
ou un sel pharmaceutiquement acceptable de ceux-ci .

6. Procédé selon la revendication 2, dans lequel ledit composé agoniste de SSTR-2 est le D-β-Nal-c[Cys-Tyr-D-Trp-Lys-Val-Cys]-Thr-NH₂ ou un sel pharmaceutiquement de celui-ci.

7. Procédé selon la revendication 2, dans lequel ledit composé agoniste de SSTR-2 est le D-Phe-Cys-Phe-D-Trp-Lys-Thr-Cys-Thr-ol ou un sel pharmaceutiquement acceptable de celui-ci.
